(19) **Europäisches Patentamt**
**European Patent Office**
**Office européen des brevets**

(11) **EP 4 625 424 A1**

(12) **EUROPEAN PATENT APPLICATION**

(43) Date of publication:
**01.10.2025 Bulletin 2025/40**

(21) Application number: **25166715.0**

(22) Date of filing: **27.03.2025**

(51) International Patent Classification (IPC):
**G16H 10/20** (2018.01)   **G06Q 50/22** (2024.01)
**G16H 40/20** (2018.01)

(52) Cooperative Patent Classification (CPC):
**G16H 10/20; G06Q 50/22; G16H 40/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2024 US 202418618584**

(71) Applicant: **Medidata Solutions, Inc.**
**New York, NY 10014 (US)**

(72) Inventor: **SOCOLOV, Alexandru**
**London, W6 7AP (GB)**

(74) Representative: **D Young & Co LLP**
**3 Noble Street**
**London EC2V 7BQ (GB)**

(54) **SYSTEMS AND METHODS TO PRODUCE A CLINICAL TRIAL SITE DISTRIBUTION MODEL**

(57)    A site distribution model identifies a set of sites to satisfy operational requirements of a clinical trial. An objective function is defined as: a first element indicating whether a site is included in the clinical trial; and a second element indicating whether a country is included in the clinical trial. There is a set of constraints including that an estimated total enrollment reaches a defined target enrollment. Computer code is generated to implement a site distribution model, using an optimization modeling language, based on the objective function and the primary set of constraints. The site distribution model is solved, if possible, to produce values of the site decision variable and the country decision variable. Otherwise, it is indicated to a user that a solution is not possible. If solving the site distribution model is possible, a list of clinical trial sites is produced from the site decision variable.

FIG. 1

**Description**

BACKGROUND

Technical Field

The present disclosure generally relates to producing a site distribution model to identify a set of sites to satisfy operational requirements of a clinical trial.

Description of the Related Art

Conventional approaches to selecting specific countries and sites for clinical trials often involve manual processes, reliance on limited data, and subjective decision-making, which can lead to several shortcomings. In some cases, existing solutions for clinical trial planning may require performing multiple iterations using unsophisticated tools, such as spreadsheets, to arrive at an acceptable distribution of clinical sites and countries. Relying on heuristics and past experiences for site selection may introduce subjectivity and potential bias into the process. In some cases, decisions may be influenced by personal preferences or anecdotal evidence rather than objective, comprehensive data analysis, leading to suboptimal site choices.

Using spreadsheets, and similar unsophisticated or informal data management and calculation tools, to manage complex data sets can lead to fragmentation and inconsistency. Information might be spread across multiple files and formats, making it difficult to get a comprehensive view of the data. This fragmentation can lead to errors in data interpretation and decision-making. Spreadsheets, especially when numerous and complex, pose challenges for effective collaboration and sharing among stakeholders involved in the site selection process. Version control issues can arise, where different team members work on different versions of a document, leading to confusion and misalignment.

As the scale of clinical trials grows, managing data and processes through spreadsheets becomes increasingly unwieldy. The manual effort required to update, analyze, and maintain spreadsheets for a large number of sites and countries is substantial and inefficient. Also, manual data entry and analysis in spreadsheets are prone to human error, and simple mistakes such as mis-keying data, incorrect formula applications, or copy-paste errors can have significant repercussions, leading to flawed analyses and decisions.

Furthermore, spreadsheets, are typically static and do not easily integrate real-time data updates. This means that the decision-making process might be based on outdated information, which is particularly problematic in the dynamic field of clinical trials where conditions and regulations can change rapidly. Moreover, while spreadsheets offer some analytical functionalities, they are limited in handling the complex, multidimensional analyses required for optimal site selection. They cannot easily perform advanced statistical analyses, predictive modeling, or scenario simulations that could provide deeper insights into the potential success of sites.

Conventional approaches may involve analyzing historical clinical site and country recruitment data and trying to come up with a scenario that mirrors a median number of sites and countries. The study planners may then manually go through site lists and pick sites based on heuristics and past experiences. This process can take weeks or months to complete and involves hundreds of hours to iterate over the myriad of potential scenarios. Thus, manual processes and traditional decision-making approaches are time-consuming and are not easily scalable to the complexities and scope of large, multi-national clinical trials. As the number of potential sites and countries increases, the ability of conventional methods to efficiently process and evaluate all necessary information diminishes. The manual nature of traditional site selection processes can result in significant inefficiencies and delays. Each site's evaluation and the coordination between different stakeholders can be slow, increasing the overall timeline of the trial setup phase. Such delays can have critical implications for the success of a trial.

Furthermore, conventional methods may not fully integrate or effectively analyze the vast amounts of data needed to make informed decisions. This includes epidemiological data, regulatory information, historical site performance, and patient demographics. Without comprehensive data integration, decisions may be based on incomplete information, leading to suboptimal site selection.

Conventional approaches often rely heavily on the experience and intuition of the decision-makers, which can introduce subjectivity and bias into the site selection process. While experience is invaluable, over-reliance on it without adequate support from objective data can lead to inconsistent and potentially biased outcomes. Moreover, human-generated scenarios are likely to be sub-optimal because one may have to choose from over 2000 relevant sites across more than 30 countries. As a practical matter, there are too many potential combinations of sites and countries for a human to accurately evaluate all possible scenarios.

Keeping up with the ever-changing regulatory landscape across different countries using conventional methods can be challenging. There is a risk of overlooking important regulatory changes or requirements, which may lead to compliance issues that could delay or jeopardize the trial. The coordination of information and decisions across various stakeholders (including sponsors, CROs, regulatory bodies, and site coordinators) can be cumbersome with traditional methods. Inefficient communication can lead to misunderstandings, misaligned objectives, and delays.

Conventional methods typically do not provide real-time decision support or adaptive planning capabilities. In the dynamic environment of clinical trials, where conditions and information can change rapidly, the inability to adapt quickly can be a significant disadvantage.

SUMMARY

Particular aspects are set out in the appended independent claims. Various optional embodiments are set out in the dependent claims.

Disclosed embodiments relate to addressing the problem of determining which sites and countries to use for performing testing of a new drug within the context of a clinical trial. More specifically, embodiments described herein outputs the number of and specific names of the sites and countries which a clinical trial needs to engage to achieve its operations parameters.

Disclosed embodiments address the problem of planning the site and country distribution for a particular clinical trial by formulating a mathematical Mixed Integer Non-Linear Program (MINLP). The objective function of the MINLP minimizes the number of sites and countries in the scenario. The constraints of the MINLP may include: reaching target enrollment, selecting a minimum number of sites per country, requiring certain countries to be present in the scenario, preserving a user-specified balance between top, medium, and low-ranked sites by historical enrollment, etc. In embodiments, the MINLP is solved using an open-source framework like COIN-OR Branch and Cut (CBC) or Basic Open-source Non-linear Mixed Integer programming (BONMIN) and the output may be presented in various forms to allow action to be taken. The output provides a list of sites and countries that satisfy all user constraints and minimize the geographic footprint of the clinical trial.

Disclosed embodiments generate a scenario in seconds rather than days which is a huge improvement in the fast-paced world of clinical trial planning.

In one aspect, the disclosed embodiments provide methods, systems, and computer-readable media to produce a site distribution model to identify a set of sites to satisfy operational requirements of a clinical trial. The method includes accessing a database of clinical trial sites. Each site is designated with a site identifier and an associated country identifier. The database comprises estimated site cumulative enrollment ($e_i$) data.

The method further includes defining an objective function. The objective function comprises finding the minimum of a sum of at least a first element and a second element. The first element includes a first weighting factor ($\alpha$) times an iterative summation of site decision variables ($z_i$), from an index value ($i$) of 1 to a total number of sites ($N$). Each of the site decision variables ($z_i$) corresponds to a site identifier and has a discrete value indicating whether a site designated by the corresponding site identifier is included in the clinical trial. The second element includes a second weighting factor ($\beta$) times an iterative summation of country decision variables ($c_j$), from an index value ($j$) of 1 to a total number of countries ($C$). Each of the country decision variables ($c_j$) corresponds to a country identifier and has a discrete value indicating whether a country designated by the corresponding country identifier is included in the clinical trial.

The method further includes receiving a primary set of constraints which must be satisfied by the site distribution model. The primary set of constraints comprises: (i) an estimated total enrollment, determined based at least in part on the estimated site cumulative enrollment ($e_i$) data, reaches a defined target enrollment; and (ii) for each site designated as included, the associated country is designated as included. The method further includes generating computer code to implement the site distribution model, using an optimization modeling language, based at least in part on the objective function and the primary set of constraints.

The method further includes solving the site distribution model, if possible, to produce values of the site decision variable and the country decision variable - otherwise indicating to a user that a solution is not possible. The method further includes producing, if the solving of the site distribution model is possible, a list of clinical trial sites from the produced values of the site decision variable.

Embodiments may include one or more of the following features, alone or in combination.

In the receiving of the primary set of constraints, the primary set of constraints may further comprise: (i) a defined maximum number of sites is not exceeded; and (ii) a defined maximum number of countries is not exceeded. The method may further include receiving a secondary set of constraints which the site distribution model seeks to satisfy, wherein the generating of computer code to implement the site distribution model is based at least in part on the objective function, the primary set of constraints, and the secondary set of constraints.

The secondary set of constraints may include a defined ratio between a number of sites in at least a first tier of sites and a number of sites in at least a second tier of sites. The at least first tier and the at least second tier may be defined based at least in part on site historical enrollment data to include, respectively, lower-ranked sites according to enrollment and higher-ranked sites according to enrollment. The secondary set of constraints may include a defined set of countries which are to be included in the clinical trial. The secondary set of constraints may include a defined minimum number of sites per country the solution must meet. The secondary set of constraints may include a defined maximum number of sites per country the solution must meet. The solving of the site distribution model may include: (i) instantiating a model object comprising the objective function, the primary set of constraints, the site decision variable, and the country decision variable; and (ii) passing the model object to a solver.

The site distribution model may include a Mixed Integer Non-Linear Program (MINLP). The optimization modeling language produces the MINLP in the form of a Python program. The indicating to the user that the solution is not possible may include indicating to the user to change one or more constraints of the primary set of constraints and the secondary set of constraints. The method may further include, if solving the site distribution model is possible, producing a projected enrollment timeline based at least in part on the list of clinical trial sites from the produced values of the site decision variable and the estimated site cumulative enrollment data.

BRIEF DESCRIPTION OF THE DRAWINGS

Fig. 1 depicts a system to produce a site distribution model to identify a set of sites to satisfy operational requirements of a clinical trial, according to disclosed embodiments.
Fig. 2 is a plot of a projected enrollment timeline based on a solution to an example site distribution model.
Fig. 3 is a plot of multiple iterations of a solution to an example site distribution model for different target enrollment times.
Fig. 4 is a plot of the optimal number of sites based on multiple iterations of a solution to an example site distribution model for different target enrollment times.
Fig. 5 depicts a method to produce a site distribution model to identify a set of sites to satisfy operational requirements of a clinical trial.

Where considered appropriate, reference numerals may be repeated among the drawings to indicate corresponding or analogous elements. Moreover, some of the blocks depicted in the drawings may be combined into a single function.

DETAILED DESCRIPTION

In the following detailed description, numerous specific details are set forth in order to provide a thorough understanding of embodiments of the presently taught approaches. However, it will be understood by those of ordinary skill in the art that the embodiments of the presently taught approaches may be practiced without these specific details. In other instances, well-known methods, procedures, components, and circuits have not been described in detail so as not to obscure the presently taught approaches.

Selecting specific countries and determining the number of sites within each country for a clinical trial is a multifaceted technical problem that requires a sophisticated technical solution due to the complexity and critical nature of the factors involved. For example, a "Phase III" clinical trial in breast cancer may require recruiting 200 patients within 24 months. It may further require using at most 180 sites across 20 countries, while using at least 5 and at most 30 sites within each country. The clinical trial may further require having sites in Brazil and Japan, while excluding any sites in China and Czechia. A further requirement may be the selection of sites, based in historical and/or estimated enrollment data, which include 25% high-ranked, 50% medium-ranked, and 25% low-ranked sites by enrollment rate. Thus, the site selection process is not arbitrary; it involves a careful balance of scientific, regulatory, logistical, and demographic considerations, each with its own set of technical challenges, as discussed in further detail below.

Different countries have varying regulatory requirements for clinical trials, governed by agencies like the Food and Drug Administration (FDA) in the United States, European Medicines Agency (EMA) in Europe, and others around the world. Navigating these regulations requires in-depth technical knowledge to ensure compliance, as failure to do so can result in significant delays, increased costs, or the invalidation of trial results. These regulatory considerations manifest as a technical constraint on an algorithmic solution to site/country selection, because the more countries there are in a clinical trial, the greater the regulatory burdens, which can result in inefficiencies and delays in the clinical trial timeline.

Identifying and accessing the right patient population is critical for the success of a clinical trial. This involves understanding the epidemiology of the disease or condition under study, including its prevalence and demographic characteristics in different regions and countries. Technical tools, data processing and storage infrastructures, and statistical methods are used to analyze demographic data and disease incidence rates to ensure that the selected sites can recruit enough eligible participants.

The capability of a particular site in a particular country to conduct a trial effectively depends on its infrastructure, personnel, and experience with clinical trials. Assessing and selecting sites involves technical evaluations of their operational capabilities, past performance, and the quality of data they produce. This might involve quantitative assessments and site audits, which are technical in nature.

The logistics of shipping trial materials, managing supply chains, and ensuring the integrity of the trial data across multiple international sites is a complex technical challenge. It requires sophisticated planning and coordination, often supported by specialized software and systems designed for clinical trial management.

Addressing these challenges typically involves a combination of advanced software tools, data analytics, simulation models, and decision-support systems. These technical solutions help in:

- Analyzing epidemiological data to identify suitable patient populations.
- Evaluating regulatory landscapes and compliance requirements across different jurisdictions.
- Assessing and selecting sites based on a range of performance and capability metrics.
- Planning and managing logistics and supply chains with the help of specialized software.
- Ensuring data integrity and standardization through EDC systems and data management protocols.

Thus, the selection of countries and sites for a clinical trial involves a complex interplay of technical considerations that require specialized knowledge, tools, and systems to address. This makes it a technical problem that necessitates a comprehensive technical solution to ensure the success of the clinical trial in terms of regulatory compliance, patient recruitment, data integrity, and overall operational efficiency.

In the examples discussed herein, a clinical trial site distribution model may be implemented as a Mixed Integer Non-Linear Program (MINLP), which is a type of mathematical optimization or decision-making problem that involves both continuous and discrete variables and has at least one non-linear element in the objective function or constraints. An MINLP problem includes:

- Objective function: a function that is to be minimized or maximized. The objective function can be non-linear and may involve both continuous and integer variables.
- Variables: the problem can include two types of variables (referred to herein as "decision variables"). Continuous variables can take any value within a given range, whereas integer variables can only take integer values within a specified range. The values of the variables that optimize the objective function (and meet the constraints and bounds) are the "solution" to the MINLP.
- Constraints: the conditions that the solution must satisfy. This can include linear and non-linear equations or inequalities involving the variables.
- Bounds: Each variable typically has upper and lower bounds defining the feasible range of values it can take.

Solving an MINLP problem involves finding the values of the continuous and integer variables that optimize the objective function while satisfying all the constraints. The non-linearities and the discrete nature of some variables can lead to complex solution landscapes with multiple local optima, making traditional optimization techniques less effective or inapplicable. Therefore, various specialized algorithms and heuristics, such as branch and bound, cutting plane methods, and metaheuristic approaches like genetic algorithms or simulated annealing, are used to tackle MINLP problems.

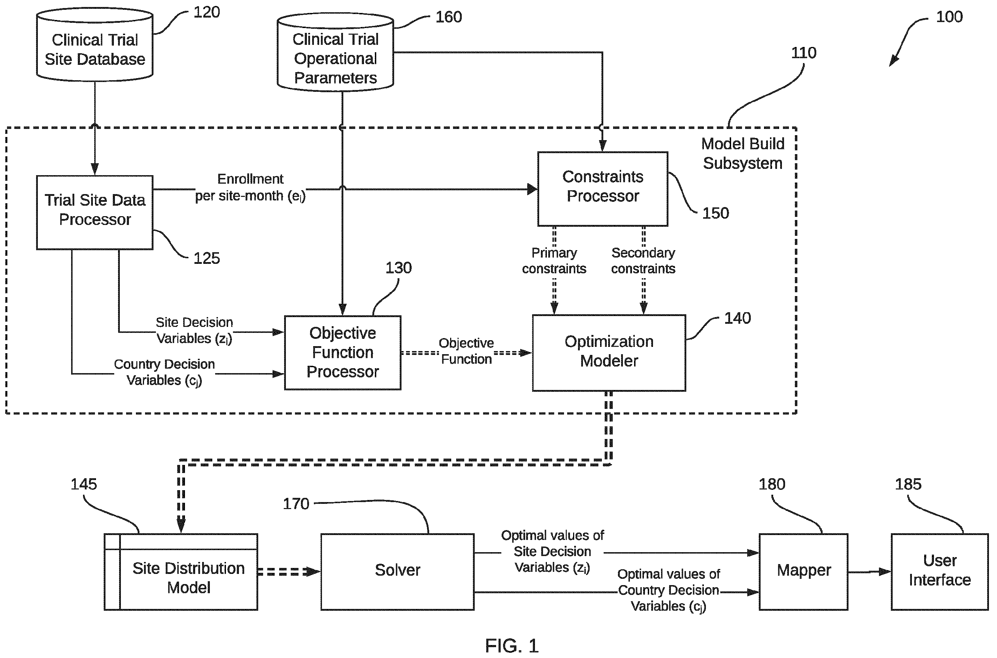

Figure 1 depicts a system 100 to produce a site distribution model 145 to identify a set of sites to satisfy operational requirements of a clinical trial. The system includes one or more computer systems or subsystems, e.g., model build subsystem 110, comprising processors and associated memory and storage. The system 100 further includes a database 120 of clinical trial sites, some portion of which may be selected for a particular clinical trial.

The database 120, which is in communication with the trial site data processor 125 includes information for each clinical trial site, including, for example, the name and location of the site, a site identifier, and an associated country identifier, as well as site-level data. In embodiments, the site-level enrollment data stored by the database 120 may include, for each site, estimated cumulative site enrollment ($e_i$) over the duration of the study, where i is an index value ranging from a value of 1 to a total number of sites (N). The estimated cumulative site enrollment ($e_i$) may be expressed in terms of the estimated cumulative number of patients enrolled by month X, where X months is a target study duration. The site-level enrollment data may be provided by various forecasting models based on historical enrollment data and/or provided by a user based on external models and datasets (see, e.g., U.S. 2023/0026758 A1).

In embodiments, the site identifier and country identifier of each site stored in the database 120 may be a numeric or alphanumeric code uniquely associated with a specific physical site situated in a specific country. In some cases, a separate identifier may not be needed if the site names and country names can be standardized within the set of clinical trial sites to ensure that they are uniquely and accurately associated with particular sites.

Based on data retrieved from the database 120, the trial site data processor 125 generates site decision variables ($z_i$), with an index (i) having values ranging from 1 to a total number of sites (N). Each of the site decision variables ($z_i$) corresponds to a site identifier and has a discrete value, e.g., a value of zero or one, indicating whether a site designated by the corresponding site identifier is included in the clinical trial site distribution. The trial site data processor 110 also generates country decision variables ($c_j$), with an index ($j$) having values ranging from 1 to a total number of countries (C). Each of the country decision variables ($c_j$) corresponds to a country identifier and has a discrete value, e.g., a value of zero or one, indicating whether a country designated by the corresponding country identifier is included in the site distribution, i.e., whether any of the sites in the site distribution are located in the particular country identified by the country identifier.

An objective function processor 130 receives the site decision variables ($z_i$) and the country decision variables ($c_j$) and applies an objective function, which finds the minimum of a sum of at least a first element and a second element:

$$\min \propto * \sum_{i=1}^{N} z_i + \beta * \sum_{j=1}^{C} c_j$$

The first element includes a first weighting factor ($\alpha$) times an iterative summation of the site decision variables ($z_i$), from an index value ($i$) of 1 to a total number of sites ($N$). Each of the site decision variables ($z_i$) corresponds to a site identifier and has a discrete value indicating whether a site designated by the corresponding site identifier is included in the clinical trial site distribution. The second element includes a second weighting factor ($\beta$) times an iterative summation of country decision variables ($c_j$), from an index value ($j$) of 1 to a total number of countries ($C$). Each of the country decision variables ($c_j$) corresponds to a country identifier and has a discrete value indicating whether a country designated by the corresponding country identifier is included in the site distribution, i.e., whether any of the sites in the site distribution are located in the particular country identified by the country identifier.

Thus, the objective function may be described as the minimum of a sum of two elements: (i) total number of sites used multiplied by a user-controlled weight ($\alpha$); and (ii) total number of countries used multiplied by a user-controlled weight ($\beta$). In embodiments, the weights $\alpha$ and $\beta$ may be retrieved from a database of clinical trial operational parameters 160. These particular parameters, $\alpha$ and $\beta$, give the user control over how "lean" or "spread-out" the geographical distribution should be. Specifically, increasing the weight (e.g., the first weighting factor, $\alpha$) on the site decision variables ($z_i$) tends to produce a solution in which the enrollment is distributed over a larger number of sites, whereas increasing the weight (e.g., the second weighting factor, $\beta$) on the country decision variables ($c_j$) tends to produce a solution in which the enrollment is distributed over a larger number of countries. The first weighting factor ($\alpha$) and the second weighting factor ($\beta$) may be determined based on the relative value of these two objectives in terms of the overall objectives of the study.

An optimization modeler 140 receives data defining the objective function from the objective function processor 130 and constraints from the constraints processor 150 based on sets of constraints input by the user. In embodiments, the user-input constraints may be retrieved from the database of clinical trial operational parameters 160.

The constraints processor 150 provides a primary set of constraints which must be satisfied by the site distribution model 145 (referred to as "always-on constraints"). Such constraints are, in effect, mandatory constraints in producing the site distribution model 145. Always-on constraints are the standard constraints in an MINLP problem that must always be satisfied by any feasible solution. These constraints are part of the problem definition and ensure that the solutions meet the necessary conditions set forth by the problem. They can be both linear and non-linear and involve any combination of the continuous and integer variables in the problem. Always-on constraints could represent physical laws, capacity limits, etc., and they define the feasible solution space by eliminating values or combinations of values that do not meet these requirements.

In embodiments, the constraints processor 150 may further provide a secondary set of constraints which the site distribution model 145 seeks to satisfy. Such constraints are, in effect, optional constraints in producing the site distribution model 145. In general, optional constraints introduce a level of conditional logic or flexibility into a problem. They are not strictly required to be satisfied by every feasible solution but can be "turned on," i.e., activated, under particular conditions, often governed by additional binary or integer variables introduced specifically for this purpose. Optional constraints can be used to model scenarios, decision-dependent situations, and conditional requirements that only apply when certain decisions are made or conditions are met.

The optimization modeler 140 generates computer code to formulate a site distribution model 145 using an optimization modeling language, based at least in part on the objective function and the primary set of constraints. In embodiments, the mathematical formulation (e.g., the objective function and the primary set of constraints) may be turned into computer code in the form of a Python program using an optimization modeling language (e.g. Pyomo). In embodiments, the implementing of the site distribution model 145 is based at least in part on the objective function, the primary set of constraints, and the secondary set of constraints.

The primary set of constraints includes the constraint that a defined target enrollment (*target_enr*) must be reached. To determine whether the target enrollment constraint has been met, an estimated total enrollment ($e_T$) is calculated based at least in part on the estimated cumulative site enrollment ($e_i$) data for the selected sites. In embodiments, the constraints processor 150 may receive the estimated cumulative site enrollment ($e_i$) data from the trial site data processor 125 and may, in turn, send it to the optimization modeler 140. Specifically, for each index value (i) for which a site decision variable ($z_i$) has a discrete value indicating that the corresponding site is included in the site distribution, the estimated cumulative site enrollment ($e_i$) for the selected site is included in a summation for all included sites to determine the estimated total enrollment ($e_T$). Thus, the target enrollment constraint may be expressed as:

$$\sum_{i=1}^{N} (e_i * z_i) \geq target\_enr$$

The primary set of constraints further includes a constraint requiring that for each site designated as included by a site decision variable ($z_i$), the associated country is designated as included by a corresponding country decision variable ($c_j$). This is necessary to ensure correspondence between included sites and the countries in which they are located. This constraint may be expressed as (wherein $in\_country_{i,j}$ indicates whether site $i$ is in country $j$):

$$z_i \leq c_j \ \ if \ \ in\_country_{i,j} == 1, \ \ i \in \{1, N\}, \ \ \ \ \ j \in \{1, C\}$$

The primary set of constraints may further include a constraint that a defined maximum number of sites is not exceeded and a constraint that a defined maximum number of countries is not exceeded. These constraints may be expressed, respectively, as follows (where $max\_sites$ is the maximum number of sites the solution may contain and $max\_countries$ is the maximum number of countries the solution may contain):

$$\sum_{i=1}^{N} z_i \leq max\_sites$$

$$\sum_{i=1}^{N} c_j \leq max\_countries$$

These constraints may be determined by the user based on the circumstances of a particular study. For example, a study may have budgetary or other limitations which require a ceiling on the number of sites. The number of countries may be limited for budgetary and/or to avoid a distribution which requires adapting to the practices of a multitude of countries, each having only a handful of sites. In some cases, these constraints may be implemented in the secondary set of constraints, which, as noted above, are not mandatory constraints. This may be the case if, for example, it is found that useful outputs can be obtained even when an ideal or practical maximum number of sites and/or countries is exceeded.

In embodiments, the secondary set of constraints may include a defined set of countries which are to be included in the clinical trial site distribution (i.e., the constraint requires at least one of the sites in the site distribution to be located in a country of the defined set of countries), a defined minimum number of sites per country, and/or a defined maximum number of sites per country. These constraints may be expressed, respectively, as follows:

$$c_j = 1 \ \ for \ \ j \in required\_countries$$

$$\frac{\sum_{i=1}^{N} in\_country_{i,j} * z_i}{min\_sites\_per\_country} \geq c_j, \ \ j \in \{1, C\}$$

$$\frac{\sum_{i=1}^{N} in\_country_{i,j} * z_i}{max\_sites\_per\_country} \leq c_j, \ \ j \in \{1, C\}$$

In embodiments, the secondary set of constraints may include a defined ratio between a number of sites in at least a first tier of sites and a number of sites in at least a second tier of sites. In embodiments, there may be, for example, three tiers of sites or more. The tiers may be defined based, at least in part, on historical site enrollment data, in which case a first tier (L) may include lower-ranked sites according to enrollment history, a second tier (M) may include medium-ranked sites according to enrollment history, and a third tier (H) may include higher-ranked sites according to enrollment history. This constraint may be expressed as follows (where *tier_ratio* is the relative ratio of t-ranked sites to be included in the solution and $in\_tier_{i,t}$ indicates whether site i is in tier t):

$$-1 < tier\_ratio_t * \sum_{i=1}^{N} z_i - \sum_{i=1}^{N} z_i * in\_tier_{i,j} < 1, \qquad t \in \{H, M, L\}$$

A solver 170 uses the site distribution model 145 to produce the values of the site decision variables ($z_i$) and the country decision variables ($c_j$). In embodiments, this may include instantiating a model object comprising the objective function, the primary set of constraints, the site decision variables ($z_i$), and the country decision variables ($c_j$). In embodiments, the model object may also comprise the secondary set of constraints. The model 145, e.g., in the form of a model object, is passed to the solver 170, which determines a solution, if a solution is possible, which includes determined values of the site decision variables ($z_i$) and the country decision variables ($c_j$). In general, a solver is an algorithm that performs a systematic search for an optimal solution, i.e., it performs particular steps and/or sequences of steps repeatedly and/or iteratively while narrowing down the solution space. In the case of a mixed-integer, nonlinear program, the solver systematically relaxes the constraints to more quickly converge on potential solutions and then attempts to enforce the constraints on the potential solutions to arrive at a solution that meets the mandatory constraints, if one exists. In some cases, there may be no solution that meets the mandatory requirements, in which case the user is directed to adjust the constraints.

A mapper 180 receives from the solver 170 the values of the site decision variables ($z_i$) and the country decision variables ($c_j$) produced in solving the site distribution model 145. As explained above, each of the site decision variables ($z_i$) corresponds to a site identifier and has a discrete value, e.g., a value of zero or one, indicating whether a site designated by the corresponding site identifier is included in the clinical trial site distribution. Each of the country decision variables ($c_j$) corresponds to a country identifier and has a discrete value, e.g., a value of zero or one, indicating whether a country designated by the corresponding country identifier is included in the site distribution.

A list of clinical trial sites is generated from the produced values of the site decision variables. The list may be displayed and/or output to the user in various forms, e.g., via a user interface 185. In embodiments, the output may include a summary of the constraints used in the generation of the solution and statistics relating to the number and location of sites and countries.

If a solution is not possible, e.g., due to the constraints being too restrictive to allow for values of the site decision variables ($z_i$) and the country decision variables ($c_j$) that solve the site distribution model 145, then the user is notified, e.g., via the user interface 180. In embodiments, such notification, i.e., indicating to the user that the solution is not possible, may include indicating to the user to change one or more constraints of the primary set of constraints and the secondary set of constraints. The user may then adjust the constraints and repeat the implementation and solving of the site distribution model 145.

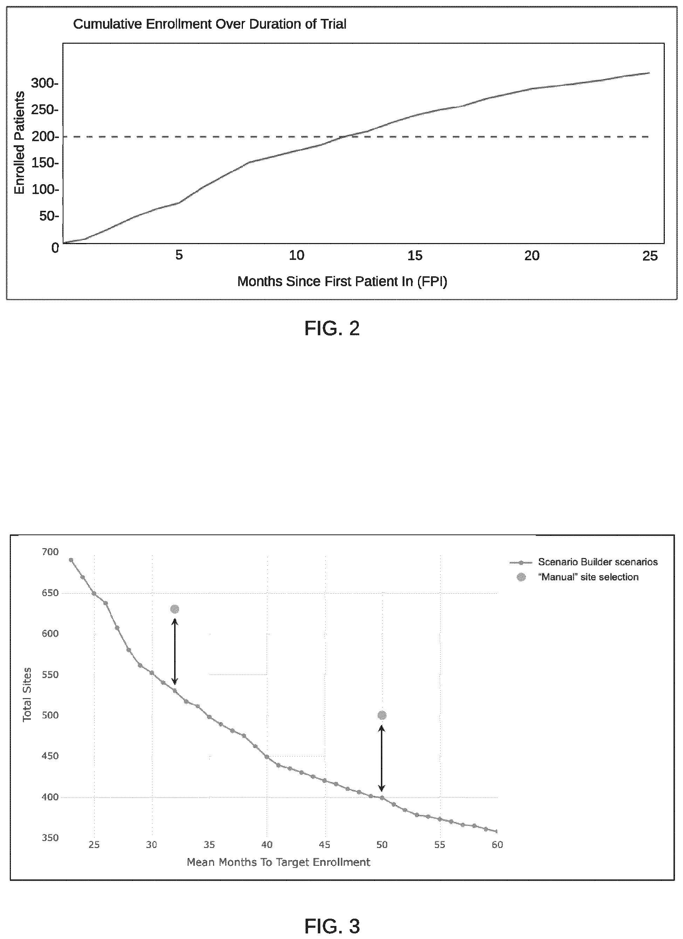

Figure 2 is a plot of a projected enrollment timeline based on a solution to a hypothetical example site distribution model. Upon obtaining a solution to the site distribution model, as discussed above, a projected enrollment timeline may be produced based at least in part on the values of the site decision variables ($z_i$) that solve the site distribution model 145. These values correspond to site identifiers of sites which are selected for inclusion in the clinical trial site distribution. The estimated total enrollment ($e_T$), i.e., total cumulative enrollment, may be calculated based at least in part on the estimated cumulative site enrollment ($e_i$) data for the selected sites.

In the example of Fig. 2, the cumulative number of enrolled patients is plotted against months since "first patient in" (FPI), i.e., when the first patient was enrolled, which is indicative of the start of the enrollment period. The dashed line corresponds to a user-specified target enrollment of 200 patients. According to this example, the projected enrollment reaches the target enrollment at around 12 months from FPI. Furthermore, other constraints were applied such as, for example, a constraint of a maximum of 150 sites and a maximum of 30 countries. A further requirement was the selection of sites, based in historical and/or estimated enrollment data, which included a certain percentage of high-ranked, medium-ranked, and low-ranked sites by enrollment rate. Another constraint was applied requiring at least three sites per selected country.

Figure 3 is a plot of the number of sites based on multiple iterations of a solution to an example site distribution model for different target enrollment times compared to conventional examples. The continuous line interconnects data points generated using techniques described herein for a specific objective function, set of constraints, etc. Each data point is for a different value of time to target enrollment. The plot indicates that as the time to target enrollment is increased, the number of sites required decreases.

The plot includes two data points for conventional approaches to selecting sites for the same underlying scenario. The first conventional data point indicates that for a target enrollment time of about 32 months, 630 sites would be selected, compared to a solution with 535 sites obtained using techniques disclosed herein. The second conventional data point indicates that for a target enrollment time of about 50 months, 500 sites would be selected, compared to a solution with 400 sites obtained using techniques disclosed herein. Thus, the disclosed approaches provide for using substantially fewer sites than conventional approaches, which would result in a significant savings in cost, time, and trial duration.

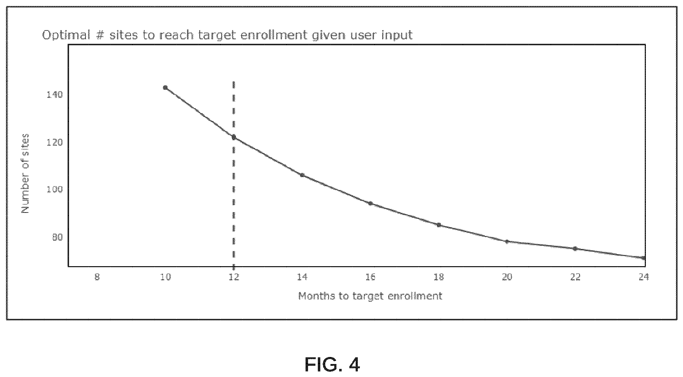

Figure 4 is a plot of the optimal number of sites based on multiple iterations of a solution to an example site distribution model for different target enrollment times compared to a specific target enrollment time. The solid line plots the number of sites versus the number of months to reach target enrollment. Each connected dot on the solid line corresponds to a scenario generated using techniques described herein. The dashed line corresponds to a hypothetical user-specified trial duration of 12 months. The same constraints were applied to this hypothetical example as in the example of Fig. 2, discussed above. The graph shows how the number of sites can be greatly reduced by extending its duration, thereby serving as a sensitivity analysis for the user-specified duration. In the example depicted, the scenario at 12 months is feasible. However, if the user had selected 8 or 10 months, then the plot would readily convey that significantly fewer sites can be used if the time to target enrollment is extended to 12 months. This provides a user with valuable guidance in selecting the clinical trial parameters.

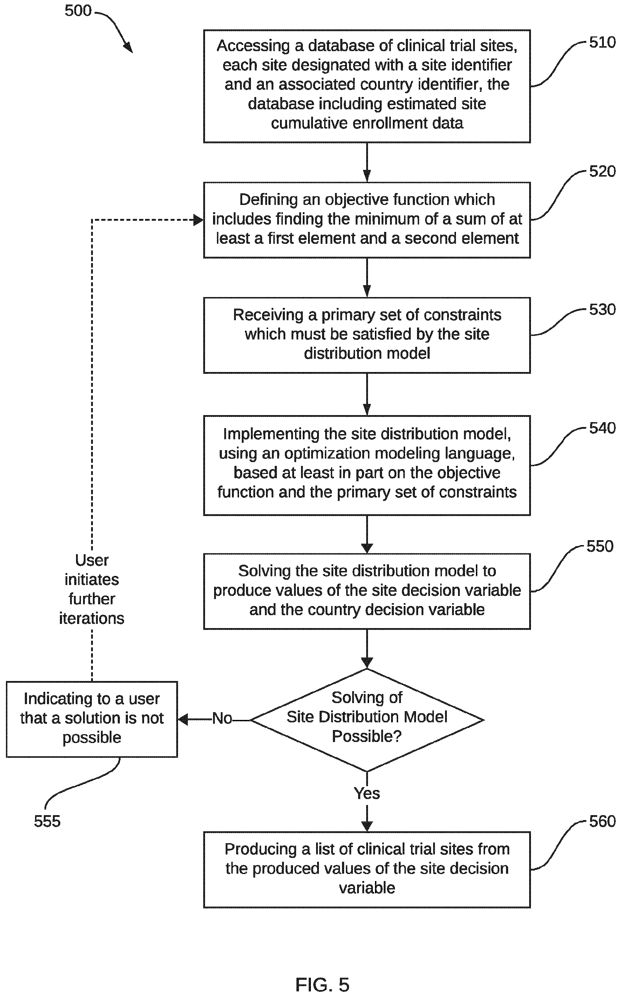

Figure 5 depicts a method 500 to produce a site distribution model to identify a set of sites to satisfy operational requirements of a clinical trial. In general, the method 500 involves translating user clinical trial parameters into a scenario with optimal sites and countries. At an initial stage, the user provides the clinical trial parameters that the output scenario will have to satisfy. For example, the user may input target patient enrollment count, regional preferences, minimum number of sites per country, etc. In embodiments, the user-input parameters are turned into a Mixed Integration Non-Linear Program (MINLP).

The user inputs are supplemented with site-level enrollment data, e.g., the number of patients enrolled by month X, where $X$ is the desired study duration. Thus, the method 500 includes accessing a database of clinical trial sites, each site designated with a site identifier and an associated country identifier (510). The database includes estimated site cumulative enrollment ($e_i$) data.

The method 500 further includes defining an objective function which includes finding the minimum of a sum of at least a first element and a second element (520). The first element includes a first weighting factor ($\alpha$) times an iterative summation of site decision variables ($z_i$), from an index value ($i$) of 1 to a total number of sites ($N$). Each of the site decision variables ($z_i$) corresponds to a site identifier and has a discrete value indicating whether a site designated by the corresponding site identifier is included in the clinical trial. The second element includes a second weighting factor ($\beta$) times an iterative summation of country decision variables ($c_j$), from an index value ($j$) of 1 to a total number of countries ($C$). Each of the country decision variables ($c_j$) corresponds to a country identifier and has a discrete value indicating whether a country designated by the corresponding country identifier is included in the clinical trial.

In embodiments, user-input parameters about a planned study may be transformed into MINLP constraints. The supported constraints may be grouped into two categories: "always-on" and "optional constraints." Thus, the method 500 further includes receiving a primary set of constraints which must be satisfied by the site distribution model (530). The primary set of constraints includes: an estimated total enrollment (determined based at least in part on the estimated site cumulative enrollment ($e_i$) data) reaches a defined target enrollment; and for each site designated as included, the associated country is designated as included. In embodiments, the primary set of constraints may further include: a defined maximum number of sites is not exceeded; and a defined maximum number of countries is not exceeded.

In embodiments, a secondary set of constraints may be received which the site distribution model seeks to satisfy, in which case the implementing of the site distribution model is based at least in part on the objective function, the primary set of constraints, and the secondary set of constraints. The secondary set of constraints may include: a defined set of countries which are to be included in the clinical trial; a defined minimum number of sites per country the solution must meet; and a defined maximum number of sites per country the solution must meet.

The method 500 further includes implementing the site distribution model, using an optimization modeling language, based at least in part on the objective function and the primary set of constraints (540). For example, the mathematical formulation (e.g., the objective function and the primary set of constraints) may be turned into a Python program using an optimization modeling language (e.g. Pyomo).

The method 500 further includes solving the site distribution model, if possible, to produce values of the site decision variables and the country decision variables (550). For example, a solver such as CBC may be used to solve the formulated MINLP program. Otherwise, if no feasible solution exists, there is an indication to the user that a solution is not possible (555). In such a case, the user may initiate further iterations and change clinical trial parameters to find a feasible solution. In embodiments, the method may involve suggesting to the user to relax constraints and/or increase trial duration.

If a feasible solution exists, the optimal selection of sites and countries (i.e., the "raw" result) is output. Thus, the method 500 further includes producing, if the solving of the site distribution model is possible, a list of clinical trial sites from the produced values of the site decision variables (560). In embodiments, the raw result may be summarized by, for example, determining the total number of sites and countries, the number of sites per country, and plotting the projected enrollment curve with this scenario. The user may review the summary assess whether there are additional parameters which were not accounted for or other shortcomings in the solution. In such a case, the user can initiate further iterations with revised clinical trial parameters. In practice, such iterations can be completed in a matter of seconds. This is in contrast to conventional approaches, which might require days or weeks to perform an iteration.

Aspects of the presently taught approaches may be embodied in the form of a system, a computer program product, or a method. Similarly, aspects of the presently taught approaches may be embodied as hardware, software, or a combination of both. Aspects of the presently taught approaches may be embodied as a computer program product saved on and/or conveyed by one or more computer-readable media in the form of computer-readable program code embodied thereon.

The computer-readable medium may be a computer-readable storage medium. A computer-readable storage medium may be, for example, an electronic, optical, magnetic, electromagnetic, infrared, or semiconductor system, apparatus, or device, or any combination thereof. A computer-readable storage medium may be described as being non-transitory in nature.

The computer-readable may also or alternatively be a transmission medium by which instructions may be conveyed. A computer-readable transmission medium may include carrier waves, transmission signals or the like. A computer-readable transmission medium may convey instructions between components of a single computer system and/or between plural separate computer systems.

Computer program code in embodiments of the presently taught approaches may be written in any suitable programming and/or scripting language. The program code may execute on a single computer, or on a plurality of computers. The computer may include a processing unit in communication with a computer-usable medium, where the computer-usable medium contains a set of instructions, and where the processing unit is designed to carry out the set of instructions, and/or a trained machine learning algorithm.

Therefore, from one perspective, there have been discussed approached for a site distribution model that identifies a set of sites to satisfy operational requirements of a clinical trial. An objective function is defined as: a first element indicating whether a site is included in the clinical trial; and a second element indicating whether a country is included in the clinical trial. There is a set of constraints including that an estimated total enrollment reaches a defined target enrollment. Computer code is generated to implement a site distribution model, using an optimization modeling language, based on the objective function and the primary set of constraints. The site distribution model is solved, if possible, to produce values of the site decision variable and the country decision variable. Otherwise, it is indicated to a user that a solution is not possible. If solving the site distribution model is possible, a list of clinical trial sites is produced from the site decision variable.

Further examples of feature combinations taught by the present disclosure are set out in the following numbered clauses.

Clause 1. A computer-implemented method to produce a site distribution model to identify a set of sites to satisfy operational requirements of a clinical trial, the method comprising: accessing a database of clinical trial sites, each site designated with a site identifier and an associated country identifier, the database comprising estimated site cumulative enrollment ($e_i$) data; defining an objective function, the objective function comprising finding the minimum of a sum of at least a first element and a second element, wherein: the first element includes a first weighting factor ($\alpha$) times an iterative summation of site decision variables ($z_i$), from an index value ($i$) of 1 to a total number of sites ($N$), wherein each of the site decision variables ($z_i$) corresponds to a site identifier and has a discrete value indicating whether a site designated by the corresponding site identifier is included in the clinical trial, and the second element includes a second weighting factor ($\beta$) times an iterative summation of country decision variables ($c_j$), from an index value ($j$) of 1 to a total number of countries ($C$), wherein each of the country decision variables ($c_j$) corresponds to a country identifier and has a discrete value indicating whether a country designated by the corresponding country identifier is included in the clinical trial; receiving a primary set of constraints which must be satisfied by the site distribution model, the primary set of constraints comprising: an estimated total enrollment, determined based at least in part on the estimated site cumulative enrollment ($e_i$) data, reaches a defined target enrollment, and for each site designated as included, the associated country is designated as included; generating computer code to implement the site distribution model, using an optimization modeling language, based at least in part on the objective function and the primary set of constraints; solving the site distribution model, if possible, to produce values of the site decision variable and the country decision variable, otherwise indicating to a user that a solution is not possible; and producing, if said solving the site distribution model is possible, a list of clinical trial sites from the produced values of the site decision variable.

Clause 2. The method of clause 1, wherein, in said receiving the primary set of constraints, the primary set of constraints further comprises: a defined maximum number of sites is not exceeded, and a defined maximum number of countries is not exceeded.

Clause 3. The method of clause 1 or 2, further comprising receiving a secondary set of constraints which the site distribution model seeks to satisfy, wherein said generating of computer code to implement the site distribution model is based at least in part on the objective function, the primary set of constraints, and the secondary set of constraints.

Clause 4. The method according to clause 3, wherein the secondary set of constraints comprises a defined ratio between a number of sites in at least a first tier of sites and a number of sites in at least a second tier of sites.

Clause 5. The method according to clause 4, wherein said at least first tier and said at least second tier are defined based at least in part on site historical enrollment data to include, respectively, lower-ranked sites according to enrollment and higher-ranked sites according to enrollment.

Clause 6. The method according to clause 3, 4 or 5, wherein the secondary set of constraints comprises a defined set of countries which are to be included in the clinical trial.

Clause 7. The method according to any of clauses 3 to 6, wherein the secondary set of constraints comprises a defined minimum number of sites per country the solution must meet.

Clause 8. The method according to any of clauses 3 to 7, wherein the secondary set of constraints comprises a defined maximum number of sites per country the solution must meet.

Clause 9. The method of any preceding clause, wherein said solving the site distribution model comprises: instantiating a model object comprising the objective function, the primary set of constraints, the site decision variable, and the country decision variable; and passing the model object to a solver.

Clause 10. The method of any preceding clause, wherein the site distribution model comprises a Mixed Integer Non-Linear Program (MINLP).

Clause 11. The method of clause 10, wherein the optimization modeling language produces the MINLP in the form of a Python program.

Clause 12. The method of any preceding clause, wherein said indicating to the user that the solution is not possible comprises indicating to the user to change one or more constraints of the primary set of constraints and the secondary set of constraints.

Clause 13. The method of any preceding clause, further comprising, if said solving the site distribution model is possible, producing a projected enrollment timeline based at least in part on the list of clinical trial sites from the produced values of the site decision variable and the estimated site cumulative enrollment data.

Clause 14. A system to produce a site distribution model to identify a set of sites to satisfy operational requirements of a clinical trial, the system comprising: a computer having one or more processors in communication with a memory, the memory storing instructions executable by said one or more processors to perform: accessing a database of clinical trial sites, each site designated with a site identifier and an associated country identifier, the database comprising estimated site cumulative enrollment ($e_i$) data; defining an objective function, the objective function comprising finding the minimum of a sum of at least a first element and a second element, wherein: the first element includes a first weighting factor ($\alpha$) times an iterative summation of site decision variables ($z_i$), from an index value ($i$) of 1 to a total number of sites ($N$), wherein each of the site decision variables ($z_i$) corresponds to a site identifier and has a discrete value indicating whether a site designated by the corresponding site identifier is included in the clinical trial, and the second element includes a second weighting factor ($\beta$) times an iterative summation of country decision variables ($c_j$), from an index value ($j$) of 1 to a total number of countries ($C$), wherein each of the country decision variables ($c_j$) corresponds to a country identifier and has a discrete value indicating whether a country designated by the corresponding country identifier is included in the clinical trial; receiving a primary set of constraints which must be satisfied by the site distribution model, the primary set of constraints comprising: an estimated total enrollment, determined based at least in part on the estimated site cumulative enrollment ($e_i$) data, reaches a defined target enrollment, and for each site designated as included, the associated country is designated as included; generating computer code to implement the site distribution model, using an optimization modeling language, based at least in part on the objective function and the primary set of constraints; solving the site distribution model, if possible, to produce values of the site decision variable and the country decision variable, otherwise indicating to a user that a solution is not possible; and producing, if said solving the site distribution model is possible, a list of clinical trial sites from the produced values of the site decision variable.

Clause 15. The system of clause 14, wherein the primary set of constraints further comprises: a defined maximum number of sites is not exceeded, and a defined maximum number of countries is not exceeded.

Clause 16. The system of clause 14 or 15, further comprising receiving a secondary set of constraints which the site distribution model seeks to satisfy, wherein said generating of computer code to implement the site distribution model is based at least in part on the objective function, the primary set of constraints, and the secondary set of constraints.

Clause 17. The system according to clause 16, wherein the secondary set of constraints comprises a defined ratio between a number of sites in at least a first tier of sites and a number of sites in at least a second tier of sites.

Clause 18. The system according to clause 17, wherein said at least first tier and said at least second tier are defined based at least in part on site historical enrollment data to include, respectively, lower-ranked sites according to enrollment and higher-ranked sites according to enrollment.

Clause 19. A computer-readable medium comprising instructions that, when executed by one or more processors of a computer, cause said one or more processors to perform a method to produce a site distribution model to identify a set of sites to satisfy operational requirements of a clinical trial, the method comprising: accessing a database of clinical trial sites, each site designated with a site identifier and an associated country identifier, the database comprising estimated site cumulative enrollment (ei) data; defining an objective function, the objective function comprising finding the minimum of a sum of at least a first element and a second element, wherein: the first element includes a first weighting factor ($\alpha$) times an iterative summation of site decision variables (zi), from an index value (i) of 1 to a total number of sites (N), wherein each of the site decision variables (zi) corresponds to a site identifier and has a discrete value indicating whether a site designated by the corresponding site identifier is included in the clinical trial, and the second element includes a second weighting factor (ß) times an iterative summation of country decision variables (cj), from an index value (j) of 1 to a total number of countries (C), wherein each of the country decision variables (cj) corresponds to a country identifier and has a discrete value indicating whether a country designated by the corresponding country identifier is included in the clinical trial; receiving a primary set of constraints which must be satisfied by the site distribution model, the primary set of constraints comprising: an estimated total enrollment, determined based at least in part on the estimated site cumulative enrollment (ei) data, reaches a defined target enrollment, and for each site designated as included, the associated country is designated as included; generating computer code to implement the site distribution model, using an optimization modeling language, based at least in part on the objective function and the primary set of constraints; solving the site distribution model, if possible, to produce values of the site decision variable and the country decision variable, otherwise indicating to a user that a solution is not possible; and producing, if said solving the site distribution model is possible, a list of clinical trial sites from the produced values of the site decision variable.

Clause 20. The computer-readable medium of clause 19, wherein the primary set of constraints further comprises: a defined maximum number of sites is not exceeded, and a defined maximum number of countries is not exceeded.

Clause 21. The computer-readable medium of clause 19 or 20, further comprising receiving a secondary set of constraints which the site distribution model seeks to satisfy, wherein said generating of computer code to implement the site distribution model is based at least in part on the objective function, the primary set of constraints, and the secondary set of constraints.

Clause 22. The computer-readable medium according to clause 21, wherein the secondary set of constraints comprises a defined ratio between a number of sites in at least a first tier of sites and a number of sites in at least a second tier of sites.

Clause 23. The computer-readable medium according to clause 22, wherein said at least first tier and said at least second tier are defined based at least in part on site historical enrollment data to include, respectively, lower-ranked sites according to enrollment and higher-ranked sites according to enrollment.

The above discussion is meant to be illustrative of the principles and various embodiments of the presently taught approaches. Numerous variations and modifications will become apparent to those skilled in the art once the above disclosure is fully appreciated. It is intended that the following claims be interpreted to embrace all such variations and modifications.

## Claims

1. A computer-implemented method to produce a site distribution model to identify a set of sites to satisfy operational requirements of a clinical trial, the method comprising:

   accessing a database of clinical trial sites, each site designated with a site identifier and an associated country identifier, the database comprising estimated site cumulative enrollment ($e_i$) data;
   defining an objective function, the objective function comprising finding the minimum of a sum of at least a first element and a second element, wherein:

   the first element includes a first weighting factor ($\alpha$) times an iterative summation of site decision variables ($z_i$), from an index value (i) of 1 to a total number of sites (N), wherein each of the site decision variables ($z_i$) corresponds to a site identifier and has a discrete value indicating whether a site designated by the corresponding site identifier is included in the clinical trial, and
   the second element includes a second weighting factor (ß) times an iterative summation of country decision variables ($c_j$), from an index value (j) of 1 to a total number of countries (C), wherein each of the country decision variables ($c_j$) corresponds to a country identifier and has a discrete value indicating whether a country designated by the corresponding country identifier is included in the clinical trial;

   receiving a primary set of constraints which must be satisfied by the site distribution model, the primary set of constraints comprising:

an estimated total enrollment, determined based at least in part on the estimated site cumulative enrollment ($e_i$) data, reaches a defined target enrollment, and

for each site designated as included, the associated country is designated as included;

generating computer code to implement the site distribution model, using an optimization modeling language, based at least in part on the objective function and the primary set of constraints;

solving the site distribution model, if possible, to produce values of the site decision variable and the country decision variable, otherwise indicating to a user that a solution is not possible; and

producing, if said solving the site distribution model is possible, a list of clinical trial sites from the produced values of the site decision variable.

2. The method of claim 1, wherein, in said receiving the primary set of constraints, the primary set of constraints further comprises:

a defined maximum number of sites is not exceeded, and
a defined maximum number of countries is not exceeded.

3. The method of claim 1 or 2, further comprising receiving a secondary set of constraints which the site distribution model seeks to satisfy, wherein said generating of computer code to implement the site distribution model is based at least in part on the objective function, the primary set of constraints, and the secondary set of constraints.

4. The method according to claim 3, wherein the secondary set of constraints comprises a defined ratio between a number of sites in at least a first tier of sites and a number of sites in at least a second tier of sites.

5. The method according to claim 4, wherein said at least first tier and said at least second tier are defined based at least in part on site historical enrollment data to include, respectively, lower-ranked sites according to enrollment and higher-ranked sites according to enrollment.

6. The method according to claim 3, 4 or 5, wherein the secondary set of constraints comprises a defined set of countries which are to be included in the clinical trial.

7. The method according to any of claims 3 to 6, wherein the secondary set of constraints comprises a defined minimum number of sites per country the solution must meet.

8. The method according to any of claims 3 to 7, wherein the secondary set of constraints comprises a defined maximum number of sites per country the solution must meet.

9. The method of any preceding claim, wherein said solving the site distribution model comprises:

instantiating a model object comprising the objective function, the primary set of constraints, the site decision variable, and the country decision variable; and
passing the model object to a solver.

10. The method of any preceding claim, wherein the site distribution model comprises a Mixed Integer Non-Linear Program (MINLP), for example wherein the optimization modeling language produces the **MINLP** in the form of a Python program.

11. The method of any preceding claim, wherein said indicating to the user that the solution is not possible comprises indicating to the user to change one or more constraints of the primary set of constraints and the secondary set of constraints.

12. The method of any preceding claim, further comprising, if said solving the site distribution model is possible, producing a projected enrollment timeline based at least in part on the list of clinical trial sites from the produced values of the site decision variable and the estimated site cumulative enrollment data.

13. A system to produce a site distribution model to identify a set of sites to satisfy operational requirements of a clinical trial, the system comprising:

a computer having one or more processors in communication with a memory, the memory storing instructions executable by said one or more processors to perform:

accessing a database of clinical trial sites, each site designated with a site identifier and an associated country identifier, the database comprising estimated site cumulative enrollment ($e_i$) data;

defining an objective function, the objective function comprising finding the minimum of a sum of at least a first element and a second element, wherein:

the first element includes a first weighting factor ($\alpha$) times an iterative summation of site decision variables ($z_i$), from an index value (i) of 1 to a total number of sites (N), wherein each of the site decision variables ($z_i$) corresponds to a site identifier and has a discrete value indicating whether a site designated by the corresponding site identifier is included in the clinical trial, and

the second element includes a second weighting factor ($\beta$) times an iterative summation of country decision variables ($c_j$), from an index value (j) of 1 to a total number of countries (C), wherein each of the country decision variables ($c_j$) corresponds to a country identifier and has a discrete value indicating whether a country designated by the corresponding country identifier is included in the clinical trial;

receiving a primary set of constraints which must be satisfied by the site distribution model, the primary set of constraints comprising:

an estimated total enrollment, determined based at least in part on the estimated site cumulative enrollment ($e_i$) data, reaches a defined target enrollment, and

for each site designated as included, the associated country is designated as included;

generating computer code to implement the site distribution model, using an optimization modeling language, based at least in part on the objective function and the primary set of constraints;

solving the site distribution model, if possible, to produce values of the site decision variable and the country decision variable, otherwise indicating to a user that a solution is not possible; and

producing, if said solving the site distribution model is possible, a list of clinical trial sites from the produced values of the site decision variable.

14. The system of claim 13, wherein said instructions are further executable by said one or more processors to perform the method of any of claims 2 to 12.

15. A computer-readable medium comprising instructions that, when executed by one or more processors of a computer, cause said one or more processors to perform the method of any of claims 1 to 12.

FIG. 1

EP 4 625 424 A1

FIG. 2

FIG. 3

Optimal # sites to reach target enrollment given user input

FIG. 4

500

Accessing a database of clinical trial sites, each site designated with a site identifier and an associated country identifier, the database including estimated site cumulative enrollment data ⌐510

Defining an objective function which includes finding the minimum of a sum of at least a first element and a second element ⌐520

Receiving a primary set of constraints which must be satisfied by the site distribution model ⌐530

Implementing the site distribution model, using an optimization modeling language, based at least in part on the objective function and the primary set of constraints ⌐540

Solving the site distribution model to produce values of the site decision variable and the country decision variable ⌐550

User initiates further iterations

Indicating to a user that a solution is not possible ←No— Solving of Site Distribution Model Possible?

555

Yes

Producing a list of clinical trial sites from the produced values of the site decision variable ⌐560

FIG. 5

Europäisches
Patentamt
European
Patent Office
Office européen
des brevets

# EUROPEAN SEARCH REPORT

**Application Number**

EP 25 16 6715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/037317 A1 (JANSSEN RES & DEVELOPMENT LLC [US]) 16 March 2023 (2023-03-16) * abstract; figures 1a, 2b * * paragraph [0003] - paragraph [0032] * * paragraph [0082] - paragraph [0095] * * paragraph [0189] - paragraph [0196] * ----- | 1-15 | INV. G16H10/20 G06Q50/22 G16H40/20 |

**TECHNICAL FIELDS SEARCHED (IPC)**

G16H
G06Q

The present search report has been drawn up for all claims

| Place of search | Date of completion of the search | Examiner |
|---|---|---|
| Munich | 5 August 2025 | Menschner, Philipp |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 6715

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2025

| Patent document cited in search report | Publication date | Patent family member(s) | Publication date |
|---|---|---|---|
| WO 2023037317 A1 | 16-03-2023 | US 2024290442 A1 | 29-08-2024 |
| | | WO 2023037317 A1 | 16-03-2023 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20230026758 A1